# EUROPEAN PATENT APPLICATION

(11) **EP 4 438 102 A1**
(43) Date of publication of application: **02.10.2024**
(21) Application number: 23166082.0
(22) Date of filing: 31.03.2023
(51) Int. Cl.: A61M 39/10, A61M 5/142

(54) **CONNECTOR FOR CONNECTING A PUMP HOSE SEGMENT TO A PUMP BED OF A PERISTALTIC PUMP**

(71) Applicant: Fresenius Medical Care Deutschland GmbH, 61352 Bad Homburg (DE)
(72) Inventor: CAVALLI, Riccardo, 61352 Bad Homburg (DE); VENERONI, Alain, 61352 Bad Homburg (DE); BRONZATO, Luca, 61352 Bad Homburg (DE)
(74) Representative: Behr, Wolfgang

(57) **Abstract**

The present invention shows a connector for connecting a pump hose segment to a pump bed of a peristaltic pump, wherein the connector comprises a first and a second fluid path associated with the ends of the pump hose segment, the first and the second fluid path crossing in the connector, wherein the connector is configured to be clipped to an opening of the pump bed by two clip elements arranged on opposite sides of the connector, characterized in that at least one of the clip elements is formed by a clip arm having a first, free end and a second, connected end connected to a main body of the connector.

## Description

The present invention relates to a connector for connecting a pump hose segment to a pump bed of a peristaltic pump.

Such a connector is known from document WO 2005/111424 A1, where the connector comprises a first and a second fluid path associated with the ends of the pump hose segment, the first and the second fluid path crossing in the connector, with the connector being configured to be clipped to an opening of the pump bed by two clip elements arranged on opposite sides of the connector.

This prior art connector is made from soft PVC, in order to provide the clip elements with the required flexibility for being clipped into the opening of the pump bed. However, by this configuration, the rigidity of the connection between the connector and the pump bed is reduced, which can in particular lead to connection problems if the pump hose segment is used for an extended time period, such as in continuous renal replacement therapy (CRRT), where a single pump segment may be continuously used for several days. Further, because the pump hose receiving sections associated with the first and the second fluid path are not arranged on the same level, the pump hose effectively has a helicoidal shape, such that the action of the pump rotor on the pump tube generates forces that act on the connector and tend to dislocate the connector from the pump bed.

These problems are already described in WO 2014/147061 A1, which therefore suggests a configuration where the connector is held in the pump bed opening by a spring arm pressing onto a top surface of the connector rather than a clip connection, and where the pump hose receiving sections are arranged on the same level to avoid forces acting on the hose. However, the configuration shown in WO 2014/147061 A1 requires a complex manufacturing process of the connector, because the connector cannot be manufactured by injection molding alone. Further, the connector requires a different configuration of the pump bed and is therefore not compatible with the prior art connectors known from WO 2005/111424 A1.

Finally, EP 2660470 B1 provides a further alternative configuration, which equally avoids the use of clip connectors and uses tubular fluid path elements forming the fluid paths, arranged in an X-configuration, as snap elements.

The object of the present invention is to provide an improved connector for connecting a pump hose segment to a pump bed of a peristaltic pump.

This object is solved by the connector according to claims 1 and 5, respectively. Preferred embodiments of the present invention are the subject matter of the dependent claims.

In a first aspect, the present invention comprises a connector for connecting a pump hose segment to a pump bed of a peristaltic pump, wherein the connector comprises a first and a second fluid path associated with the ends of the pump hose segment, the first and the second fluid path crossing in the connector, wherein the connector is configured to be clipped to an opening of the pump bed by two clip elements arranged on opposite sides of the connector. The first aspect is characterized in that at least one of the clip elements is formed by a clip arm having a first, free end and a second, connected end connected to a main body of the connector.

By this configuration, the flexibility of the clip arm can be controlled by the shape of the clip arm and its connection to the main body, allowing for a more rigid and secure connection also under prolonged use.

In particular, the clip arm may form a spring element that pushes against the pump stator, in particular to one of the pump bed end sections. Thereby, a correct fixation of the connector and ease of installation is ensured. Further, the connection is not affected by temperature and pump speed. In particular, a material can be used that is not prone to material softening, even over prolonged use.

In an embodiment of the present invention, the clip arm is extending freely from the connected end to the free end, and/or not connected to the main body of the connector at a position between the free end and the connected end.

In an embodiment of the present invention, the clip arm is formed by a plate element connected on one end to the main body of the connector, with a main surface of the plate element extending in parallel to the axis of rotation of the rotor of the peristaltic pump and/or perpendicular to a plane defined by the fluid paths and the pump hose segment connected thereto, the plate element extending from the connecting end to the free end in a direction perpendicular to the axis of rotation of the rotor of the peristaltic pump and/or in parallel to a plane defined by the fluid paths and the pump hose segment connected thereto.

In an embodiment of the present invention, the ratio of the length of the clip arm measured from the connecting end to the free end along its extension in a direction perpendicular to the axis of rotation of the rotor of the peristaltic pump and/or in parallel to a plane defined by the fluid paths and the pump hose segment connected thereto with respect to their height measured in a direction in parallel to the axis of rotation of the rotor of the peristaltic pump and/or perpendicular to a plane defined by the fluid paths and the pump hose segment connected thereto, is at least 2:1, preferably at least 3:1.

In an embodiment of the present invention, the connector has a first and a second pump hose receiving section associated with the first and a second fluid path for respectively receiving a first and a second end of the pump hose segment.

In particular, the pump hose receiving sections may be tubular elements into which the ends of the pump hose segment are inserted.

In an embodiment of the present invention, the connected end of the clip arm is connected to one of the pump hose receiving sections of the connector. In particular, the clip arm may be formed by a plate-like element connected to and surrounding the pump hose receiving section, such that an end of the pump hose segment passes through an opening in the plate-like element into a tubular element forming the pump hose receiving section and being arranged on a back-side of the plate-like element. In an embodiment of the present invention, both clip elements are formed by clip elements having a second, connected end connected to a main body of the connector.

Preferably the connected ends of both clip elements are respectively connected to one of the pump hose receiving sections of the connector. In particular, a first clip element may be connected to a first pump hose receiving section and a second clip element may be connected to a second pump hose receiving section.

Further, both clip elements may have a middle section that extends separately from the main body.

Whether the first end of the second clip element is a free end or a connected end will depend on the embodiment.

In a first embodiment of the present invention, both clip elements are formed by clip arms having a first, free end and a second, connected end connected to a main body of the connector. Preferably, in this case, both clip arms are only connected to the main body at their connected end.

Further, all the features described above with respect to the at least one clip arm preferably apply to both clip arms, in particular the features relating to the plate-like form of the clip arms and how they are respectively connected to one of the pump hose receiving sections of the connector.

In a second embodiment, only one of the clip elements is formed as a clip arm having a free end as defined above, while the other clip element is connected to a main body of the connector at both ends.

In particular, the other clip element may be connected with a second connected end to a pump hose receiving section of the connector, and with a first connected end to a connecting hose receiving sections of the connector.

In an embodiment, the other clip element may be formed by a plate element connected on a second connecting end to the main body of the connector, with a main surface of the plate element extending in parallel to the axis of rotation of the rotor of the peristaltic pump and/or perpendicular to a plane defined by the fluid paths and the pump hose segment connected thereto, the plate element extending from the second connecting end to the first connecting end in a direction perpendicular to the axis of rotation of the rotor of the peristaltic pump and/or in parallel to a plane defined by the fluid paths and the pump hose segment connected thereto.

In an embodiment of the present invention, the ratio of the length of the other clip element measured from the first connecting end to the second connecting end along its extension in a direction perpendicular to the axis of rotation of the rotor of the peristaltic pump and/or in parallel to a plane defined by the fluid paths and the pump hose segment connected thereto with respect to their height measured in a direction in parallel to the axis of rotation of the rotor of the peristaltic pump and/or perpendicular to a plane defined by the fluid paths and the pump hose segment connected thereto, is at least 2:1, preferably at least 3:1.

In an embodiment of the present invention, the other clip element is not connected to the main body of the connector between the first connecting end and the second connecting end.

In an embodiment of the present invention, the other clip element may be formed by a plate-like element connected to and surrounding the pump hose receiving section with the second connecting end, such that an end of the pump hose segment passes through an opening in the plate-like element into a tubular element forming the pump hose receiving section and being arranged on a back-side of the plate-like element, and/or connected to and surrounding the connecting hose receiving section with the first connecting end, such that an end of the connecting hose segment passes through an opening in the plate-like element into a tubular element forming the connecting hose receiving section and being arranged on a back-side of the plate-like element.

In an embodiment of the present invention, the other clip element may have a third, free end provided on the clip element at a position between the first and the second connected element. In particular, the free end may be formed by a plate-like element connected to the plate-like elements forming the clip element between the first and the second connected element.

In an embodiment of the present invention, the third free end and the second connected element may connect to an end section of the pump bed opening.

In an embodiment of the present invention, the other clip element does not have a third, free end, but is only formed by a single arm extending between the two connected ends.

In an embodiment of the present invention, the other clip element is connected to the end section of the pump bed at a position between the two connected ends.

Preferred embodiment of both the first embodiment having only one clip arm and the second embodiment having two clip arms are discussed in the following:
In an embodiment of the present invention, the clip elements and/or clip arms are shaped to match the shape of the end segments of the pump bed to which they are to be connected.

Therefore, the at least one clip arm is or the two clip arms are shaped to match the shape of the end segments of the pump bed to which they are to be connected.

In an embodiment of the present invention, a first clip element has a rounded shape and a second clip element has an angled shape.

Therefore, if there is only one clip arm, the clip arm may have a rounded shape or an angled shape, preferably a rounded shape.

In particular, the other clip element may have an angled shape or a round shape.

In an embodiment, the third free end and the second connected end are connected to each other at an angle, forming the angled shape of the other clip element.

In an embodiment, the clip element has the angled shape or the round shape in a middle portion extending between the two connected ends.

If there are two clip arms, the first clip arm has a rounded shape and a second clip arm has an angled shape.

In an embodiment of the present invention, one of the clip elements or clip arms, preferably a first clip element or clip arm having a rounded shape, is provided with a connecting element for connecting to an ejection pin and/or a sensor of the peristaltic pump. The connecting element may be provided on a protrusion provided on a back-side of the clip element or clip arm and/or may be directed to face a bottom side of the pump bed.

In a second, independent aspect the present invention comprises a connector for connecting a pump hose segment to a pump bed of a peristaltic pump, wherein the connector comprises a first and a second fluid path associated with a first and a second end of the pump hose segment, respectively, the first and the second fluid path crossing in the connector, wherein the first fluid path forms an inlet fluid path and the second fluid path forming an outlet fluid path, the connector having a bottom side configured to face a bottom of the pump bed, wherein the connector has a first and a second pump hose receiving section associated with the first and a second fluid path for respectively receiving the first and the second end of the pump hose segment, and wherein the connector has a first and a second connecting hose receiving section associated with the first and a second fluid path for respectively receiving an inlet connecting hose segment and an outlet connecting hose segment. The second aspect is characterized in that the second fluid path crosses below the first fluid path on the bottom side of the connector and the second pump hose receiving section is positioned closer to the bottom side of the connector than the first pump hose receiving section.

This configuration of the outlet fluid path of the connector passed below the inlet fluid path and the second pump hose receiving section receiving an outlet side of the pump hose segment is positioned closer to the bottom side of the connector than the first pump hose receiving section receiving an inlet side of the pump hose segment. The configuration of the outlet fluid path and the inlet fluid path is therefore inverted in the present invention with respect to the prior art configurations discussed above. The inventors of the present invention have found that surprisingly, by this remarkably simple solution, the problems associated with the helicoidal configuration of the pump hose discussed above for the prior art configuration can be avoided, because even though there is still a helicoidal configuration, the forces generated by the rotor of the pump now act in a direction towards the bottom side of the pump bed and therefore do not loosen the connection of the connector to the pump bed even after prolonged use.

The first and the second aspect of the present invention are independently from each protected by the present file.

For example, the second aspect could also be used in a connector that does not have clip arms, but is clipped to the pump bed opening by other forms of clip elements, for example clip elements connected to the main body of the connector at both ends.

In a preferred embodiment, the two aspects are combined in a single connector.

Embodiments of the present invention, which discuss further features applicable both to a connector according to the fist aspect, to a connector according to the second aspect, and their combination will be discussed in the following.

In an embodiment of the present invention, a main body of the connector is formed by two tubular fluid path elements comprising the first and a second fluid path, connected to each other where the tubular fluid path elements cross each other, wherein each of the tubular fluid path elements comprises a pump hose receiving section on one end and a connecting hose receiving section on the opposite end. This configuration reduces the material necessary for the production of the connector and its weight.

In an embodiment of the present invention, the fluid path elements are only connected to each other where they cross each other.

In an embodiment of the present invention, the fluid path elements are connected to each other where they cross each other, and in addition, the connecting hose receiving section of a first fluid path element and the pump hose receiving section of a second fluid path element are connected by a clip element.

In an embodiment of the present invention, at least one of the connecting hose receiving sections and/or at least one of the pump hose receiving sections is formed by a free end of the fluid path elements.

In a first embodiment of the present invention, the connecting hose receiving sections and/or the pump hose receiving sections are each formed by free ends of the fluid path elements.

In a second embodiment, the connecting hose receiving section of a second fluid path element and the pump hose receiving section of a first fluid path element are formed by free ends of the respective fluid path elements.

In an embodiment of the present invention, the connector comprises a third fluid path connected to the first or the second fluid path and a third connecting hose receiving section associated with the third fluid path for connecting a third connecting hose to the first or the second fluid path via the third fluid path. The third fluid path can for example be used for heparin infusion. The third connecting hose may for example be connected to a heparin pump for this purpose.

In an embodiment of the present invention, the third fluid path is provided by a third tubular fluid path element connected to the first or second tubular fluid path element with a first end and preferably comprising the third connecting hose receiving section on its second end.

In an embodiment of the present invention, the third tubular fluid path element extends in parallel to the second or first tubular fluid path element, preferably with a distance therebetween.

In an alternative embodiment of the present invention, the third tubular fluid path and the second or first tubular fluid path element are formed in a single tubular element.

In an embodiment of the present invention, the connector is formed by injection molding as a single, integral piece.

In an embodiment of the present invention, the clip arms and/or the connector is formed from a plastics material having a shore hardness D of at least D 60, preferably of at least 70, further preferably at least D 75. By using a relatively hard plastic, the stiffness and the connection properties of the connector can be improved.

In the first aspect, due to the use of the clip arms, the clip functionality is nevertheless ensured as the clip arms are flexible due to their shape and one-sided connection to the main body, such that the use of a soft plastic is no longer required.

In an embodiment of the present invention, the clip arms and/or the connector is formed from a plastics material having a shore hardness D of at maximum D 90, preferably at maximum D 85.

Shore hardness D is preferably measured as defined in ASTM D2240-00 or DIN EN ISO 868.

In an embodiment of the present invention, the clip arms and/or the connector is formed from PVC, in particular hard PVC.

The present invention further comprises a tubing set comprising a connector as defined above according to the first and/or second aspect, a pump hose segment and two connecting hose segments connected to the connector.

In an embodiment of the present invention, the tubing set is a medical tubing set, in particular a disposable medical tubing set.

In an embodiment of the present invention, the tubing set is a blood tubing set comprising a connector for connecting to a dialyzer provided at one of the connecting hose segments, preferably an outlet connecting hose segment.

The present invention further comprises a peristaltic pump comprising a stator with a pump bed formed therein, a pump hose segment inserted into the pump bed in arcuate form, a rotor for acting on the pump hose segment, and a connector as defined above according to the first and/or second aspect.

The peristaltic pump may in particular be a pump for pumping medical liquids.

The present invention further comprises peristaltic pump comprising a stator with a pump bed formed therein, a pump hose segment inserted into the pump bed in arcuate form, a rotor for acting on the pump hose segment, and a connector for connecting the pump hose segment to the pump bed, wherein the connector comprises a first and a second fluid path associated with a first and a second end of the pump hose segment, respectively, wherein the first fluid path forms an inlet fluid path and the second fluid path forming an outlet fluid path, the first and the second fluid path crossing in the connector, the connector having a bottom side facing a bottom of the pump bed. The connector has a first and a second pump hose receiving section associated with the first and a second fluid path for respectively receiving the first and the second end of the pump hose segment. The peristaltic pump comprises a main pump operation mode where the rotor rotates in a first direction to move along the pump hose segment in a direction from the first end to the second end of the pump hose segment. According to the second aspect of the present invention, the second fluid path crosses below the first fluid path on the bottom side of the connector and the second pump hose receiving section is positioned closer to the bottom side of the connector than the first pump hose receiving section.

The main pump operation mode may be used during a treatment performed by the pump, such as a dialysis treatment.

The present invention further comprises a medical device, in particular a dialysis machine, comprising at least one pump as described above.

In an embodiment of the present invention, the medical device comprises a controller configured to automatically control the at least one pump during a treatment performed by the medical device to operate in the main operation mode.

The present invention is now described with respect to preferred embodiments and drawings.

The drawings show:
- Fig. 1: A top view of a first embodiment of the inventive connector,
- Fig. 2: a perspective view of the first embodiment shown in Fig. 1 ,
- Fig. 3: a side view from the pump bed side on the first embodiment shown in Figures 1 and 2,
- Fig. 4: an embodiment of an inventive peristaltic pump comprising the first embodiment of a connector and corresponding hose sections, with the pump hose segment being connected to the pump bed of the peristaltic pump by the connector,
- Fig. 5: the situation shown in Fig. 4 in perspective view, schematically showing the fluid flow directions and the force imparted on the connector by the pumping action,
- Fig. 6: a perspective view of a second embodiment of an inventive connector,
- Fig. 7: a sectional view of a second fluid flow path of the second embodiment shown in Fig. 6,
- Fig. 8: a sectional view showing the first and the third fluid flow path of the second embodiment shown in Fig. 6, and
- Fig. 9: a third embodiment of a connector,
- Fig. 10: a fourth embodiment of a connector in a perspective view,
- Fig. 11: the fourth embodiment in a top view,
- Fig. 12: a fifth embodiment of a connector in a perspective view,
- Fig. 13: the fifth embodiment in a top view.

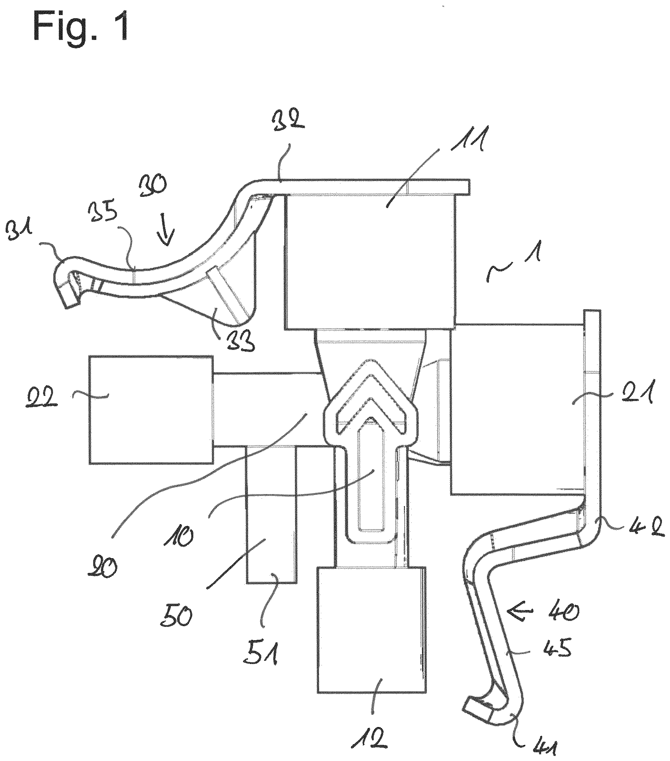

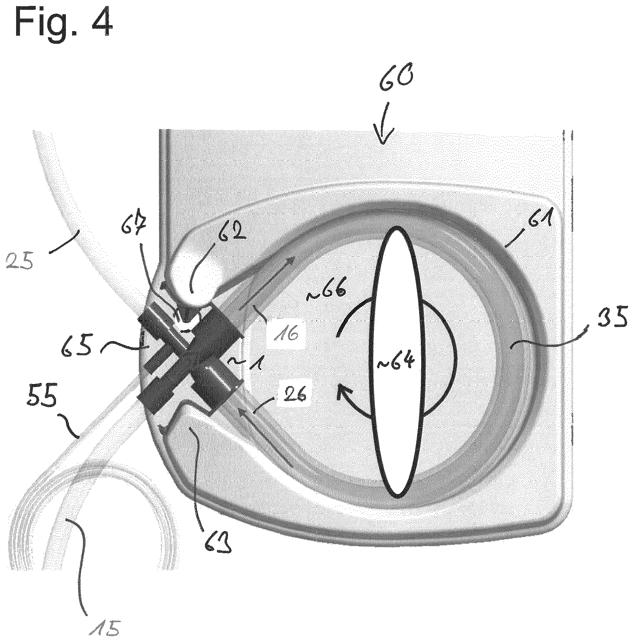

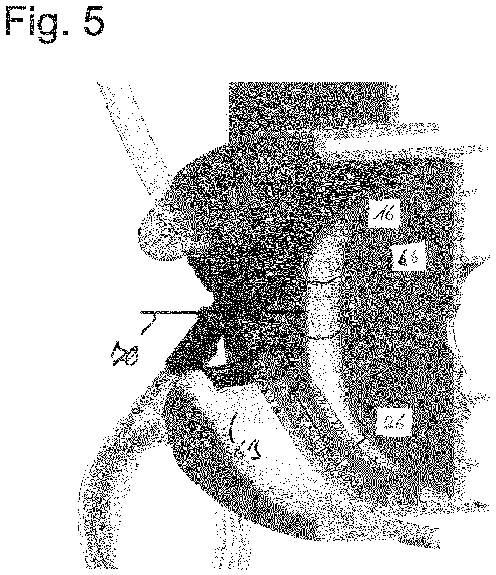

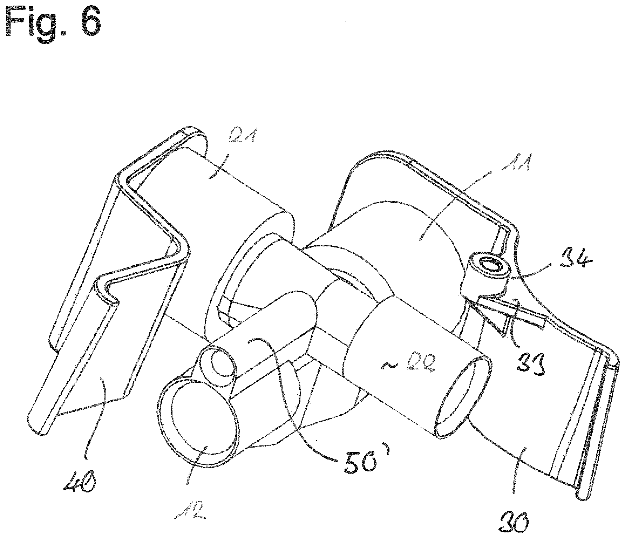

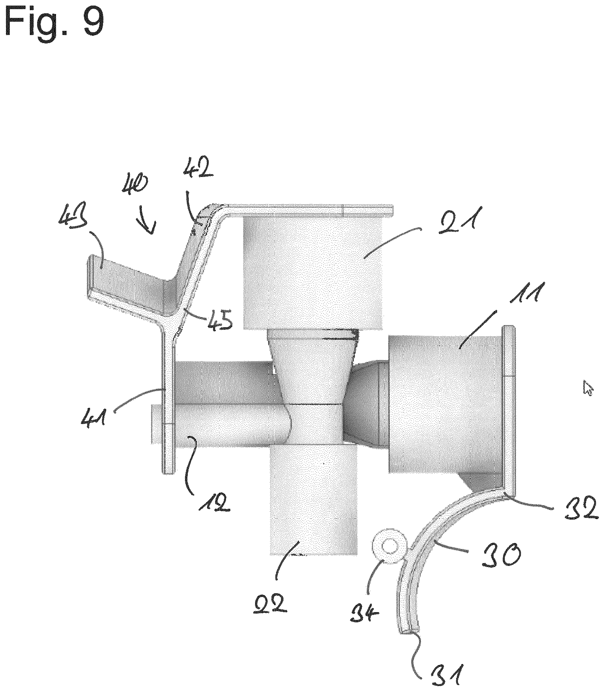

Figures 1 - 3 show a first embodiment of a connector according to the present invention, and Figures 4 and 5 show the use of the inventive connector for connecting a pump hose segment 35 to a pump bed 61 of a peristaltic pump 60. Figures 6 to 8 show a second embodiment, which however differs from the first embodiments only with respect to the third fluid path, such that all the remaining discussion refers equally to both embodiments. Figures 9, 10 and 11 as well as 12 and 13 show a third to fifth embodiment, respectively, which differs from the first or second embodiment with respect to the configuration of the first or second clip element, which in this case has a second connected end connected to the main body, but all the remaining discussion also refers to the third to fifth embodiment.

In the peristaltic pump as shown in Fig. 4, the pump hose segment is inserted into the pump bed 61 in arcuate form to form a pump loop, with the ends of the rotor 64, schematically drawn in Fig. 4, acting on the pump hose segment. By the action of the ends of the rotor 64, the pump hose segment is compressed between the rotor and the pump bed 61, thereby closing the flow path inside the pump hose segment at the respective positions. Thereby, by rotation of the rotor 64, fluid is pumped along the pump hose segment from an inlet site 16 of the pump hose segment to an outlet site 26 of the pump hose segment.

The peristaltic pump 61 may for example be a roller pump, with rollers being provided on the ends of the rotor 64 for acting on the pump hose segment.

The connector 1 of the present invention is configured to releasably connect the pump hose segment to the pump bed 61. In particular, the pump bed 61 has a pump bed opening 65 provided on one side, with the connector 1 being inserted between the end segments 62 and 63 of the pump bed forming the side walls of the pump bed opening.

The connector 1 comprises a first fluid path 10 and a second fluid path 20 associated with a first end 16 of the pump hose segment 35 and the second end 26 of the pump hose segment 35, respectively, with the fluid paths crossing within the connector. The first and second ends 16 and 26 of the pump hose segment are respectively connected to one end of the first and second fluid paths 10 and 20 of the connector. To the opposite ends of the fluid paths 10 and 20, connecting pump hose segments 15 and 25 are connected.

In particular, the first fluid path 10 of the connector 1 is provided with a first pump hose receiving section 11 for receiving the first end 16 of the pump hose segment 35, and a first connecting hose receiving section 12 for receiving a connecting hose segment 15, which in the present configuration is an inlet connecting hose segment. Further, the second fluid path 20 is provided with a second pump hose receiving section 21 for receiving the second end 26 of the pump hose segment and a second connecting hose receiving section 22 for receiving the connecting hose 25, which in the present case is an outlet connecting hose.

Further, in the embodiment shown, the connector is provided with a third fluid path 50 connected to the second fluid path 20. The third fluid path 50 is provided with a third connecting hose receiving section 51 for receiving a further connecting hose 55, which may for example be used for heparin infusion. The third fluid path is however an optional feature.

According to the first aspect of the present invention, the connector is configured to be clipped to the opening 65 of the pump bed by two clip elements arranged on opposite sides of the connector 1, with at least one or both of the clip elements being formed by a clip arm 30, 40 having a first free end 31, 41, and a second, connected end 32, 42, connected to the main body of the connector 1. In particular, the at least one clip arm may only be connected to the main body with the connected end 32, 42, with the remaining clip arm extending separately from the main body to the free end 31, 41.

By this configuration, a more reliable and stable connection between the connector 1 and the pump bed opening can be provided then with prior art configurations. In particular, because at least one of the clip elements are formed as clip arms, due to their shape and their connection to the main body on one side of the clip arms only, they will allow a controlled deformation of the clip arms with respect to the main body, and therefore a secure connection to the opening 65 of the pump bed.

In all the embodiments, both clip elements 30 and 40 have a second, connected end by which they are connected to the main body. Further, they have a middle section 35, 45 that extends separately from the main body. Whether the first end is a free and, such that the clip element is a clip arm, or a connected end, depends on the configuration.

In the first and second embodiment shown in Fig. 1 to 8, both clip elements 30 and 40 are formed as clip arms having a first free end and a second connected end, with the middle section 35, 45 extending there between.

In the third to fourth embodiment, one of the clip elements 30, 40 is formed as clip arm having a first free end and a second connected end, with a middle section 35, 45 extending there between, and the other clip element 40, 30 has a first and a second connected end with a middle section 45, 35 extending there between.

Further, in these embodiments, the first connected end of the connection element having two connected ends is connected to a connecting hose receiving section.

In the third embodiment shown in Fig. 9, the first clip element 30 is formed as a clip arm having a first free end 31 and a second connected end 32. The second clip element 40 has two connected ends 41 and 42, respectively connected to the main body of the connector. In particular, the first connected end 41 is connected to the connecting hose receiving section 12 of the first fluid path element 10 and the second connected end 42 is connected to the pump hose receiving section 11 of the first fluid path element 10. Further, the clip element 30 has a third, free end 43, connected to the clip element to the middle section 45 extending between the two connected ends 41 and 42, the free end 43 extending towards the outside.

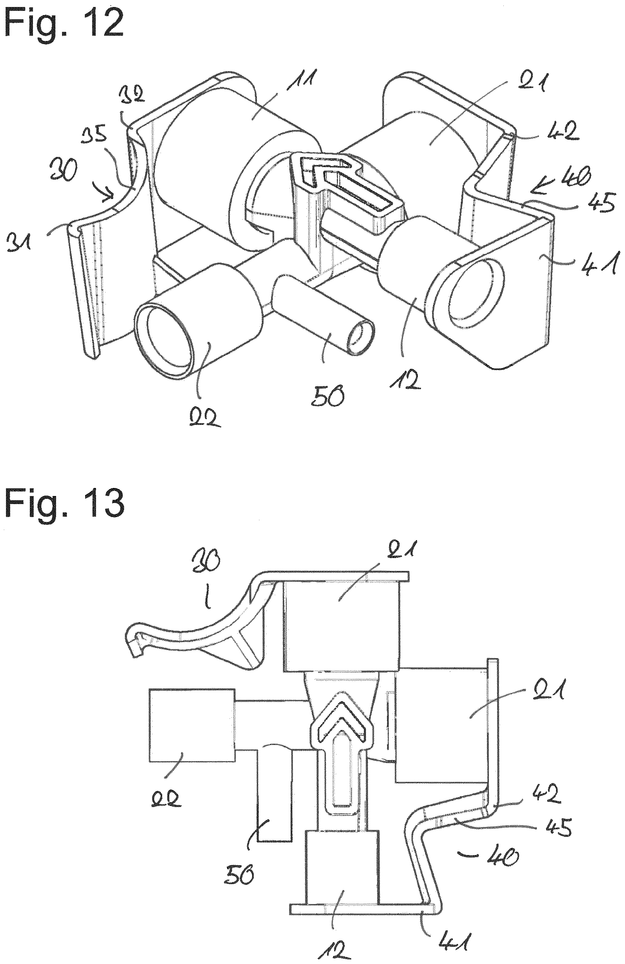

In the fifth embodiment shown in Fig. 12 and 13, the first clip element 30 is formed as a clip arm having a first free end 31 and a second connected end 32. The second clip element 40 has two connected ends 41 and 42, respectively connected to the main body of the connector. However, in this embodiment, the second clip element 40 is not provided with an additional third, free end, but has only the middle section 45 that extends from the first connected end 41 to the second connected end 42.

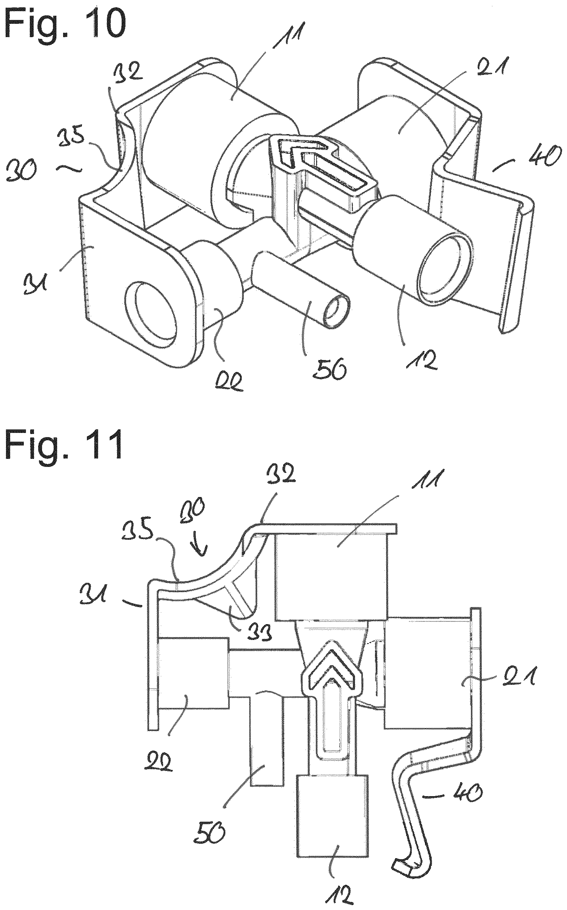

In the fourth embodiment shown in Fig. 10 and 11, it is the second clip element 40 that is formed as a clip arm having a first free end 41 and a second connected end 42. The first clip element 30 has two connected ends 31 and 32, respectively connected to the main body of the connector. As in the fifth embodiment, the first clip element 30 having two connected ends is not provided with an additional third, free end, but has only the middle section 35 that extends from the first connected end 31 to the second connected end 32.

The clip elements or arms 30, 40 essentially have the shape of plate elements extending from their connection with the main body of the connector to their free end or the first connected end and/or the third free end, with the shape of the plate elements matched to the contour of the end sections 62 and 63 of the pump bed 61 to which they connect, and a main surface of the plate elements extending in parallel to the axis of rotation of the rotor. In particular, the clip elements or clip arms abut to the end sections 62 and 63 of the pump bed 61 at least with a part of the middle section 35, 45, such that at least with a part of the middle section matches the contour of the end sections 62 and 63 of the pump bed 61 at least with a part of the middle section.

In all the embodiments, the first clip arm or clip element 30 has a rounded shape, while the second clip element or clip arm 40 has an angled shape. The round and the angled shape both fit to a corresponding round and angled shape of the tube bed end sections 62 and 63 to which they are connected. In particular, they reach around the tube bed end sections 62 and 63.

As can be seen in particular from figure 5, the pump bed end sections 62 and 63 each have wall sections essentially extending in parallel with an axis of rotation of the rotor 64, to which the clip arms or elements 30 and 40 connect. Further, the pump bed end sections 62 and 63 are provided, on their upper ends, with protrusions protruding from the side walls and extending above the clip arms or clip elements, such that the connector is held by the shape of the clip arms or clip elements extending around the wall sections of the pump bed end sections 62 and 63 in the plane being formed by the pump hose segment, and are held by the protrusions in a directions perpendicular thereto, i.e. in a direction in parallel with the axis of rotation of the rotor.

For inserting the connector in the pump bed opening, the at least one clip arm is therefore deformed to pass around the protrusions until the clip arms or clip elements are firmly seated below the protrusions.

As shown e.g. in figures 1 - 3, the clip arms or elements 30 and 40 are connected with their second connected ends 32 and 42 to the first and second pump hose receiving sections 11 and 21, respectively.

In the embodiment, if the clip element is formed as a clip arm, this connection is the only connection of the clip arms to the main body, such that the clip arms can be deformed relative to the pump hose receiving sections due to the flexibility of the clip arms at their connected ends 32, 42 and along their extension from the connected ends to the free ends 31, 41.

In the embodiment shown in Fig. 9 and 12, 13, this applies to the first clip arm 30, while the second clip element 40 is connected to the main body also at its second end 41, such that the main flexibility for the clip connection is provided by the first clip arm. In the embodiment shown in Fig. 10, 11, this applies to the second clip arm 40, while the first clip element 30 is connected to the main body also at its second end 31, such that the main flexibility for the clip connection is provided by the second clip arm

The clip arm 30 and/or 40 is or are therefore wing-shaped spring arms that can be deformed relative to the main body for insertion into the pump bed opening.

In the embodiment shown in Fig. 9, the angled form of the second clip element 40 is provided by a part of the middle section 45 extending from the second connecting end 42 and by the third free end 43, which are connected to each other at an angle. The abutment to the angled end section of the pump bed is therefore provided by the second clip element 40 between the second connecting end 42 and the third free end 43. The first connecting end 41 extends from the connecting hose receiving section 12 to a position where the middle section 45 extending from the second connecting end 42 and the third free end 43 are connected at an angle.

In the fourth and fifth embodiments, the first connecting end 31, 41 is connected to what is the outer end of the third free end 43 in Fig. 9, such that the second connection element is not provided with a free end.

In particular, in the fourth and fifth embodiments, the clip element 30, 40 that is not formed as a clip arm extends from the first connected ends 31, 41 via the middle section 35, 45 comprising the entire contour that is connected to the respective end section of the pump bed to the second connected end 32, 42, respectively. Therefore, in Fig. 10 and 11, the middle section 35 has a rounded shape, and in Fig. 12 and 13, the middle section 45 has an angled shape.

Further features relating to all embodiments are described in the following.

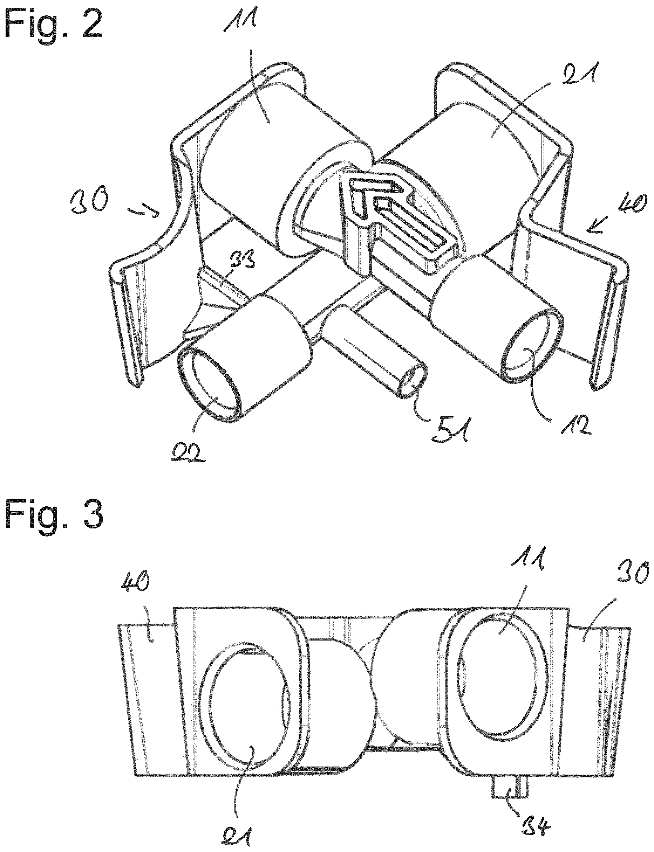

As can be seen from figures 2 and 3 as well as 9, the plate-like structure forming the clip arms and/or elements 30 and 40 extends around the pump hose receiving sections, such that the opening provided by the pump hose receiving sections for receiving the ends of the pump hose segment 35 are provided as holes within the plate-like structures that extend from the pump hose receiving sections to the outside to form the clip arms and/or elements.

Further, in all the embodiments, the middle section 35, 45 is formed by a plate element.

The plate-like shape of the clip arms and/or elements essentially extends in parallel to an axis of rotation of the rotor, and has a shape following the contour of the pump bed end segments 62 and 63 in the plane formed by the pump hose segment, extending perpendicular to the axis of rotation of the rotor.

As can be further seen in particular in figures 2 and 4 and 9, the first clip arm 30 is provided with a connecting element 34 provided on an element 33 projecting on the back side of the clip arm 30. The connecting element 34 extends to a bottom side of the connector and connects to an ejection pin and/or a sensor of the peristaltic pump.

This ejector 67 is shown in figure 4, and generates an upward force on the first clip arm 30 for ejecting the connector from the pump bed opening. Further, the connector 34 may equally be used in order to sense the presence of the connector within the pump bed opening.

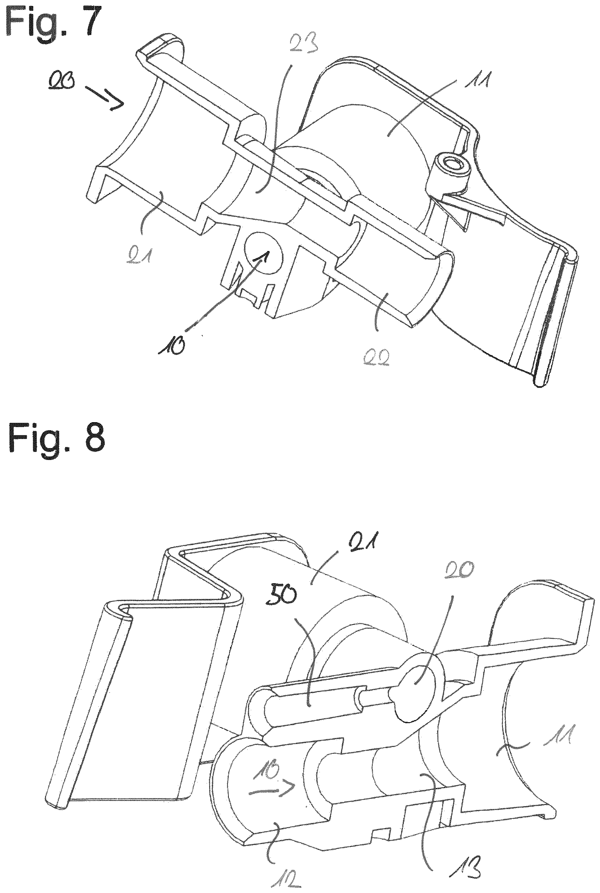

The shape of the connector 34 can for example be seen in figures 6 and 7 showing the second embodiment, which in this respect is identical to the first aspect. In particular, the connector has the shape of a cylinder extending in parallel to an axis of the rotor 64 when connected to the pump bed, with a pin of the ejection actor and/or sensor being received in an opening of the connection element 34 from below.

According to a second aspect of the present invention, the second fluid path 20, which forms an outlet fluid path for the pump hose segment, passes the first fluid path 10 on a bottom side of the connector, which forms an inlet fluid path. Further, the second pump hose receiving section 21 is positioned closer to the bottom side of the connector 1 than the first pump hose receiving section 11, see in particular figure 3. This means that when the connector is connected to the pump bed, the second pump hose receiving section 21 is positioned, when measured in an axial direction along the axis of the rotor, closer to the bottom side of the pump bed than the first pump hose receiving section 11, the bottom side of the connector being the side of the connector facing the bottom 66 of the pump bed when inserted into the pump bed opening.

By this configuration, the pump hose segment connected to the pump hose receiving sections 11 on an inlet side and 21 on an outlet side still has a helicoidal form. However, contrary to the prior art solutions, the outlet side 26 of the pump hose is provided closer to the bottom of the pump bed than the inlet side 16 of the pump hose segment, such that the helicoidal form of the pump hose moves to the bottom side of the pump bed in the pumping direction. Thereby, the vertical forces generated by the action of the rotor on the pump hose segment act in a direction 70 indicated in figure 5 towards the bottom side of the pump bed, rather than to a top side and therefore away from the pump bed as in prior art solutions. By this simple inversion in the relative position of the inlet and outlet fluid paths 10 and 11 with respect to the prior art solution, the long-term stability of the connection can be decisively improved by the second aspect of the present invention.

In this respect, we note that while it would in principle be preferred to arrange the two pump hose receiving sections on the same level with respect to an axial direction of the pump, in order to avoid the helicoidal form altogether. This would however require a very complex manufacturing process of the connector, because the fluid paths then could no longer be formed by injection molding.

In particular, as can be seen from figures 7 and 8, showing the connector from a bottom side with sections along the first fluid path 10 and the second fluid path 20 for the second embodiment, which in this aspect, however, is identical to the first embodiment, if one fluid path passes below the other fluid path without a bend in the fluid paths, this necessarily leads to a certain axial displacement of the pump hose receiving sections 11 and 21. However, a fluid path with a bend cannot be formed by injection molding. The present invention according to the second aspect therefore retains the different axial positions of the pump hose receiving sections, but avoid the problems associated therewith, as explained above.

The shape of the fluid paths inside the connector, which equally applies to a connector according to the first aspect, will now be further described with respect to Fig. 7 and 8.

As can be seen from figures 7 and 8, the pump hose receiving sections 11 and 21 have a larger diameter than the connecting hose receiving sections 12 and 22, but extend in parallel thereto. Between the pump hose receiving sections and the connecting hose receiving sections, the first and second fluid path each have a connecting passage 13, 23 that has a straight extension and a continuously decreasing diameter or crosssection at least over a part of its extension.

However, the connecting hose receiving sections are not arranged collinear with the respective pump hose receiving sections, but displaced thereto to the bottom or top side.

In particular, for the second fluid path 20 crossing on the bottom side, the connecting hose receiving section 22 is arranged such that its center line is moved to the bottom side with respect to the center line of the pump hose receiving section 21, such that the side wall of the connection passage 23 has a straight shape on the bottom side, while it has a taper shape on the top side. For the fluid path 10 passing on the top side, the center line of the connecting hose receiving section 12 is respectively positioned away from a center line of the respective pump hose receiving section 11 to the top side, such that a wall section of the connecting passage 13 has a straight extension on a top side and a taper shape on the bottom side.

Because of this configuration and because the connecting passages 13 and 23 cross at a position where the diameter is already reduced, the axial displacement between the pump hose receiving section 11 and 21 is reduced as in comparison with a configuration where the pump hose receiving sections would be collinear with the connecting hose receiving sections. Still, as can be seen from figure 3, there is a certain axial displacement leading to the helicoidal configuration.

The first and the second aspect of the present invention are in principle independent from each other. However, preferably and in the embodiment shown, they are combined in the connector of the present invention.

In a third aspect of the invention, which can be combined first with the first and/or the second aspect, the main body of the connector 1 is formed by two tubular fluid path elements respectively comprising the first and the second fluid path 10 and 20, connected to each other where the tubular fluid path elements cross each other. Therefore, the main body is essentially formed by two tubular elements crossing each other.

As can be seen from Figures 1 - 5, in the embodiment, the tubular fluid path elements are only connected where they cross each other, and are not included in a housing or provided with a bottom plate to which they would be connected. In the embodiment shown in Fig. 9, the tubular fluid path elements are connected where they cross each other, and the pump hose receiving section 21 of the second fluid path element 20 is connected to the connecting hose receiving section 12 of the first fluid path element 10 by the clip element 40. However, also in this embodiment, the tubular fluid path elements are not provided with a bottom plate to which they would be connected. Thereby, the material needed for the manufacture of the connector, as well as its weight, can be reduced.

By the configuration of the main body being formed by two tubular fluid path elements, at least one or both of the connecting hose receiving sections 12 and 22 are each formed by free ends of such a tubular fluid path element. Further, at least one or both of the pump hose receiving sections 11 and 21 are equally formed by opposing free ends of a tubular fluid path element.

In the embodiment shown in figures 1 - 3 as well as 10 to 13, the third fluid path 50 is provided by a third tubular fluid path element 51 connected to a second tubular fluid path element forming the second fluid path, and extending in parallel to the first tubular fluid path element forming the first fluid path, but with a distance thereto. Therefore, in the first, fourth and fifth embodiment, the third fluid path 50 is equally formed by a tubular fluid path element separate from the first tubular fluid path element and connected to the second tubular fluid path element 21 at a position between the crossing and the connecting hose receiving section 22. Due to the distance between the third and the first tubular fluid path element, the connection of the respective hoses to the respective connecting hose receiving sections is simplified.

The second embodiment shown in Figures 6 - 8 is identical in all aspects to the first embodiment, but for the position and configuration of the third fluid path. In particular, in the second embodiment, the tubular fluid path element forming the third fluid path directly adjoins the second tubular fluid path element forming the second fluid path, such that both fluid paths extend in a single, common tubular element. Further, the third fluid path 50 is connected to the first fluid path 10 at the position where it crosses the second fluid path 20.

In the third embodiment shown in Fig. 9, the third fluid path element is configured as in the second embodiment. In a further alternative embodiment, the third fluid path element of the connector of Fig. 9 could also be configured as in the first embodiment, i.e. separately from the first fluid path element, as in the fourth and fifth embodiment. In a preferred configuration, the connector of the present invention is formed by injection molding as a single, integral piece.

Preferably, the connector is formed by a single type of plastics material.

The clip arms and/or the connector is preferably formed by a plastics material having a shore hardness D of between D60 and D90, preferably between D70 or D75 and D85. For example, the clip arms and/or the connector may be formed from hard PVC, for example having a shore hardness D of D 77.

Using a hard plastic material improves the rigidity of the connector and therefore the connection with the pump bed.

The pump hose receiving section and the connecting hose receiving section are each formed by tubular sections into which the respective ends of the hoses are inserted. Further, the inner diameter of the connecting passages 13 and 23 is respectively chosen such that the inner surface of the respective hoses form a continuous surface with the inner surface of the connecting passage 13 and 23.

The tubing set comprising the connector, the pump hose segment and the connecting hose segments may in particular be a medical tubing set. In particular, the tubing set may be an extracorporeal tubing set. In an embodiment, it may be a blood tubing set comprising a dialyzer or a connector for connecting the tubing set to a dialyzer. In particular, the connector for the dialyzer may be provided on outlet connecting hose segment. The pump may equally be used for pumping dialysate.

The inventive peristaltic pump may for example be part of a medical device, such as a dialysis machine.

The pump and/or the medical device may comprise a controller configured to control the pump, during a treatment operation, to rotate in a first rotating direction in order to move fluid from the inlet side 16 of the pump hose segment to the outlet side 26 of the pump bed hose.

The dialysis machine may in particular be a hemodialysis machine or a machine for Continuous Renal Replacement Therapy (CRRT).

Further, the dialysis machine may be provided with two or more peristaltic pumps, one for pumping blood, the other for pumping dialysate or other medical fluids, each provided with a connector and a pump hose segment as described above.

## Claims

1. A connector for connecting a pump hose segment to a pump bed of a peristaltic pump,
wherein the connector comprises a first and a second fluid path associated with the ends of the pump hose segment, the first and the second fluid path crossing in the connector,
wherein the connector is configured to be clipped to an opening of the pump bed by two clip elements arranged on opposite sides of the connector, **characterized in that**
at least one of the clip elements is formed by a clip arm having a first, free end and a second, connected end connected to a main body of the connector.

2. The connector of claim 1, wherein the connector has a first and a second pump hose receiving section associated with the first and a second fluid path for respectively receiving a first and a second end of the pump hose segment, wherein preferably the connected end of the clip arm is connected to one of the pump hose receiving sections of the connector.

3. The connector of one of the preceding claims, wherein both clip elements are formed by clip arms having a first, free end and a second, connected end connected to a main body of the connector, wherein preferably the connected ends of both clip arms are respectively connected to one of the pump hose receiving sections of the connector.

4. The connector of one of the preceding claims, wherein a first clip element has a rounded shape and a second clip element has an angled shape,, and/or wherein one of the clip elements, preferably a first clip element having a rounded shape, is provided with a connecting element for connecting to an ejection pin and/or a sensor of the peristaltic pump.

5. A connector for connecting a pump hose segment to a pump bed of a peristaltic pump,
wherein the connector comprises a first and a second fluid path associated with a first and a second end of the pump hose segment, respectively, the first and the second fluid path crossing in the connector, wherein the first fluid path forms an inlet fluid path and the second fluid path forming an outlet fluid path, the connector having a bottom side configured to face a bottom of the pump bed,
wherein the connector has a first and a second pump hose receiving section associated with the first and a second fluid path for respectively receiving the first and the second end of the pump hose segment,
wherein the connector has a first and a second connecting hose receiving section associated with the first and a second fluid path for respectively receiving an inlet connecting hose segment and an outlet connecting hose segment,
**characterized in that**
the second fluid path crosses below the first fluid path on the bottom side of the connector and the second pump hose receiving section is positioned closer to the bottom side of the connector than the first pump hose receiving section.

6. The connector of one of the preceding claims, wherein a main body of the connector is formed by two tubular fluid path elements comprising the first and a second fluid path, connected to each other where the tubular fluid path elements cross each other, wherein each of the tubular fluid path elements comprises a pump hose receiving section on one end and a connecting hose receiving section on the opposite end.

7. The connector of claim 6, wherein the fluid path elements are only connected to each other where they cross each other, and/or wherein at least one and preferably both of the connecting hose receiving sections and/or at least one and preferably both of the pump hose receiving sections are each formed by a free end of the fluid path elements.

8. The connector of one of the preceding claims, comprising a third fluid path connected to the first or the second fluid path and a third connecting hose receiving section associated with the third fluid path for connecting a third connecting hose to the first or the second fluid path via the third fluid path.

9. The connector of claim 8, wherein the third fluid path is provided by a third tubular fluid path element connected to the first or second tubular fluid path element and preferably comprising the third connecting hose receiving section on its free end, wherein the third tubular fluid path element preferably extends in parallel to the second or first tubular fluid path element, preferably with a distance therebetween.

10. The connector of one of the preceding claims, wherein the connector is formed by injection molding as a single, integral piece.

11. The connector of one of the preceding claims, wherein the clip arms and/or the connector is formed from a plastics material having a shore hardness D of at least D 60, preferably at least D 70, further preferably at least D 75 and/or having a shore hardness D of at maximum D 90, preferably at maximum D 85 and/or wherein the clip arms and/or the connector is formed from PVC, in particular hard PVC.

12. A tubing set comprising a connector according to one of the preceding claims, a pump hose segment and two connecting hose segments connected to the connector, wherein the tubing set is preferably a blood tubing set comprising a connector for connecting to a dialyzer provided on one of the connecting hose segments, preferably an outlet connecting hose segment.

13. A peristaltic pump comprising
a stator with a pump bed formed therein,
a pump hose segment inserted into the pump bed in arcuate form,
a rotor for acting on the pump hose segment, and
a connector according to one of the preceding claims.

14. A peristaltic pump comprising
a stator with a pump bed formed therein,
a pump hose segment inserted into the pump bed in arcuate form,
a rotor for acting on the pump hose segment, and
a connector for connecting the pump hose segment to the pump bed, wherein the connector comprises a first and a second fluid path associated with a first and a second end of the pump hose segment, respectively, wherein the first fluid path forms an inlet fluid path and the second fluid path forming an outlet fluid path, the first and the second fluid path crossing in the connector, the connector having a bottom side facing a bottom of the pump bed,
wherein the connector has a first and a second pump hose receiving section associated with the first and a second fluid path for respectively receiving the first and the second end of the pump hose segment,
wherein the peristaltic pump comprises a main pump operation mode where the rotor rotates in a first direction to move along the pump hose segment in a direction from the first end to the second end of the pump hose segment,
**characterized in that**
the second fluid path crosses below the first fluid path on the bottom side of the connector and the second pump hose receiving section is positioned closer to the bottom side of the connector than the first pump hose receiving section.

15. A medical device, in particular a dialysis machine, comprising a pump according to one of claims 13 or 14.
